# EUROPEAN PATENT SPECIFICATION

(11) **EP 3 982 646 B1**
(45) Date of publication and mention of the grant of the patent: **17.07.2024**
(21) Application number: 20200680.5
(22) Date of filing: 08.10.2020
(51) Int. Cl.: H04R 25/00, H04W 12/08, G16H 10/60, H04L 9/40

(54) **PROTECTED SHARING OF DATA SAVED IN A HEARING DEVICE**
GESCHÜTZTE GEMEINSAME NUTZUNG VON IN EINEM HÖRGERÄT GESPEICHERTEN DATEN
PARTAGE PROTÉGÉE DE DONNÉES ENREGISTRÉES DANS UN DISPOSITIF AUDITIF

(43) Date of publication of application: 13.04.2022
(73) Proprietor: Sonova AG, 8712 Stäfa (CH)
(72) Inventor: El Guindi, Nadim, 8303 Bassersdorf (CH); Thielen, Anne, 8712 Stäfa (CH); Breitenmoser, Andreas, 8004 Zürich (CH); KRUEGER, Harald, 8910 Affoltern am Albis (CH)
(74) Representative: Sykora & König Patentanwälte PartG mbB

(56) References cited:
- WO-A1-2018/091079
- US-A1- 2013 142 367
- US-A1- 2019 191 255
- US-A1- 2020 034 553
- US-A1- 2020 273 566

## Description

### TECHNICAL FIELD PRESENT TECHNOLOGY

The present technology relates to a method, a computer program and a computer-readable medium for a protected sharing of data saved in a hearing system, which comprises at least one hearing device used by a hearing device user, with a third party device. Furthermore, the present technology relates to a hearing system comprising at least one hearing device used by a hearing device user and optionally a connected user device, such as a smartphone.

### BACKGROUND

Hearing devices are generally small and complex devices. Hearing devices can include a processor, microphone, speaker, memory, housing, and other electronical and mechanical components. Some example hearing devices are Behind-The-Ear (BTE), Receiver-In-Canal (RIC), In-The-Ear (ITE), Completely-In-Canal (CIC), and Invisible-In-The-Canal (IIC) devices. A user can prefer one of these hearing devices compared to another device based on hearing loss, aesthetic preferences, lifestyle needs, and budget.

In many cases, modern hearing devices include sensors, e.g. accelerometers, pulse oximetry or electroencephalography sensors etc., in order to collect health related data of the user, such as data about his physical activity or hear rate. Such data is typically stored in the hearing device and may contain sensitive or private information about the physical or mental state of the user.

In certain situations, especially if a medical condition is diagnosed, it may be desirable to share the data stored in a hearing device with a health care professional. At the same time, the data should not be accessible by unauthorized parties, such as a hacker working for a life insurance company.

To protect a hearing device against various types of hacking, inter alia against unauthorized reading out of health data saved in the hearing device, EP 3 236 674 A1 discloses a hearing device provided with public key security functions. That is, a part of the hearing device of EP 3 236 674 A1 is configured as a so-called Trusted Platform Module including a secure crypto processor, a random generator, as well as a protected memory for private keys, which is only readable by the crypto processor, but not by the main processor. The public key of the device, the public key of a hearing device manufacturer and an operating system are saved in a storage region of a non-volatile memory which is protected against modification, i.e. in a sealed storage region.

Further, WO 2018/091079 A1 discloses a method of controlling access of a client to a service of a hearing instrument, comprising the steps of: requesting access of the client to the service of the hearing instrument by providing a client authenticator to the hearing instrument; authenticating the client based on a validation of the provided client authenticator by the hearing instrument; upon successful authentication, comparing a security level associated with the service requested by the client with a highest security level assigned to the client by the hearing instrument, and granting access if the requested security level is below or equal to the highest security level assigned to the client. The client may comprise devices such as fitting stations, hearing instruments, wireless microphones, smartphones, tablets, remote controls etc. Since the authentication methods include, for example, authentication by user gesture, the user keeps control of client access to his hearing instrument. Further, the method protects the hearing instrument from man-in-the-middle attacks during pairing, while nevertheless the access control may be implemented in a manner that requires only little resources of the hearing instrument.

US 2020/273566 A1 discloses ear-wearable devices configured to selectively export particular types of source data to a computing system. The computing system may enable third-party user to access health-related data belonging to a sharable health-related data type, provided that third-party user is a user (e.g., person, organization, group of persons and/or organizations) allowed by user to access the health-related data belonging to the sharable health-related data type,

US 2020/034553 A1 discloses multi-party consent for accessing medical data. One example use case is the so called "Four-Eyes Policy" according to which two human actors of sufficient rank must provide consent for a specific target object, or object group, within a specific time window.

### DESCRIPTION PRESENT TECHNOLOGY

It is an objective of the present technology to further improve the protection of data saved in a hearing system, e.g. sensitive health data of the user saved in his hearing device, so that even serious hackers may not be able to access this data. It is a further objective of the present technology to enable a secure sharing of the data saved in a hearing system with authorized third parties, such as physicians, who should have access to this data.

These objectives are achieved by the subject-matter of the independent claims. Further exemplary embodiments are evident from the dependent claims and the following description.

A first aspect of the present technology relates to a method for a protected sharing of data saved in a hearing system, which includes at least a hearing device used by a hearing device user, with a third party device. In general, a third party may be any person or organization other than the hearing device user. For example, the third party may be one or more of the following group of persons or organizations: a health or medical professional; a hearing care professional; a general practitioner; a medical specialist; a call center; a hearing care manufacturer, an emergency service or the public, who might need some data saved in the hearing device, such as health data of the hearing device user. A third party device may, for example, be a personal computer or a smartphone of the third party.

The method may be a computer-implemented method, which may be performed automatically by the hearing system. The hearing system as described herein below may be adapted for performing the method. The hearing system may, for instance, comprise one or two hearing devices used by the same user. One or both of the hearing devices may be worn on and/or in an ear of the user. A hearing device may be a hearing aid, which may be adapted for compensating a hearing loss of the user. Also a cochlear implant may be a hearing device. The hearing system may optionally further comprise at least one connected user device, such as a smartphone, smartwatch, tablet or other devices in particular bluetooth enabled, carried by the user.

According to an embodiment of the present technology, the method comprises: storing a definition of a first trusted party and of a second trusted party in the hearing system. Beside storing such a definition in a hearing device, this may also include storing a definition in a connected user device, such as a smartphone, and/or in a central server connected with the hearing device in a predefined way. A connected user device and/or the central server may be regarded as parts the hearing system.

The first trusted party and the second trusted party may, for example, be two different persons or organizations from the following group: the hearing device user himself; a health professional; a hearing care professional; a significant other, such as a spouse or another family member or other person close to the hearing device user; a hearing device manufacturer.

According to an embodiment of the present technology, the method further comprises: providing (e.g. transmitting or directing) a sharing request for the data to be shared to a device of the first trusted party (e.g. to a smartphone of the hearing device user) and to a device of the second trusted party (e.g. to a smartphone or personal computer of the significant other). The sharing request may, for instance, be generated by the user of the hearing device, using his smartphone or the hearing device. Alternatively, e.g. in an emergency case, the sharing request may also be generated by the third party, such as an emergency service.

According to an embodiment of the present technology, the method further comprises: generating a first sharing approval by the device of the first trusted party and a second sharing approval by the device of the second trusted party, and sharing the data with the third party device only upon generation of the first and the second sharing approval, i.e. only if both sharing approvals have been successfully generated. The above-mentioned step of storing a definition of the first trusted party and of the second trusted party in the hearing system may, as the case may be, also include storing a definition of a respective type of generation of a sharing approval or a computer-implemented algorithm of generating or validating the respective sharing approval.

In other words, according to the present method, at least two different predefined parties - the first and the second trusted party (and, as the case may be, a number of further trusted parties) whose definition is saved in the hearing system and/or a server connected to the hearing system - must authorize the sharing of data saved in the hearing system with a third party. If at least one of the sharing approvals of the trusted parties defined in the system is missing or fails, the sharing of data with the third party device will be prohibited and will not be performed. In this manner, the sharing of data saved in the hearing system can be reliably protected.

According to an embodiment of the present technology, different trusted parties may be authorized for different kinds of sharing approvals. A sharing approval may, for example, be restricted to a predetermined type of data to be shared, e.g. only authorized for approving the sharing of pulse data, but not of diagnosis data. Furthermore, respective sharing approvals may be restricted with respect to the receiver, i.e. the intended third party, e.g. only authorized for approving the sharing of data with medical practitioners, but not with insurance companies.

The sharing approvals may, as the case may be, provided with a time limit of their validity, so that data cannot be shared after the time limit, e.g. one month after data generation and/or after initiating the sharing request, has expired. It also may be that there is a spatial limit, i.e. that data sharing is only allowed at home, for example.

According to an embodiment of the present technology, the data to be shared is collected by a sensor which is a part of the hearing device and/or it may be derived from such sensor data (e.g. diagnostic data). It may be that one or more different sensors are integrated in the hearing device and/or transmit data measured at some other body part of the hearing device user by wireless communication to the hearing device.

In this sense, hearing devices provided with sensors may, for example, collect and store sensitive health data of one or more of the following types: pulse; body temperature; blood pressure; blood oxygen; blood sugar; electroencephalography (EEG) data; audiograms; falls and balance disorders; Dementia, Alzheimer's, Parkinson related data; Corona related data, e.g. concerning contact tracing, test, and vaccination.

The sensors may comprise one or more of the following: an air conduction microphone; a bone conduction microphone; an optical sensor; an electrical sensor; a heart-rate sensor; a blood pressure sensor; an electrocardiography (ECG), electroencephalography (EEG) or electrooculography (EOG) sensor.

However, the presence of a data collecting sensor is not indispensable for the present technology. For example, at least a part of the data to be shared may also be useful data manually saved in the hearing device, e.g. by a hearing care professional or by the hearing device user.

According to an embodiment of the present technology, the method further comprises: storing a definition of at least one further trusted party in the hearing system; providing a sharing request to a device of each further trusted party; and generating a further sharing approval by the device of each further trusted party. In this case, the data will be shared with the third party device only upon generation of the first sharing approval, the second sharing approval, and the at least one further sharing approval, i.e. only if further sharing approvals have been generated by the respective devices of all of the further trusted parties, too. By further increasing the number of trusted parties, the security provided by the present method may be further improved. All the details described above and in the following with respect to the first or the second trusted parties may pertain to each of the further trusted parties as well.

According to an embodiment of the present technology, storing a definition of the respective trusted party comprises storing its public key in the hearing system; and providing the sharing request to the device of the respective trusted party comprises encrypting the data to be shared with the public key of the respective trusted party and transmitting the encrypted data to its device. In this embodiment, the generation of the respective sharing approval by the device of the respective trusted party comprises decrypting the data to be shared with a private key of the respective trusted party and, for example, transmitting thus decrypted data to a predetermined receiving device of the hearing system for a next step of the present method.

With this embodiment, for example, a cascaded encryption may be implemented in that the data to be shared is encrypted with the public keys of several trusted parties and, as the case may be, additionally also with a public key of the third party. Thus encrypted data may be, first, transmitted to the device of the first trusted party, who can prove all the necessary details and approve the sharing by decrypting the data in its device with its private key and by transmitting thus decrypted data to the device of the second trusted party. The second trusted party can proceed in the same manner and transmit the data decrypted with its private key in its device to the device of a further trusted party or, if no further trusted parties are predefined in the hearing system, to the device of the third party, thus sharing the data with the third party. This may be seen as a four-eyes principle implemented by the method.

In this or any other embodiment which makes use of asymmetric encryption including a pair of keys, i.e. a public and a private key, alternatively, symmetric encryption may be implemented in the present method and system. The solution based on symmetric encryption may comprise the step of exchanging the symmetric key based on asymmetric encryption and/or based on a public key infrastructure. Further, in any embodiment described herein above or in the following which makes use of a public key, alternatively or in addition, a certificate including predefined additional information about the respective party may be used, for a still higher security of the method. The certificate may comprise a time limit.

Furthermore, any embodiment described herein above or in the following, which makes use of asymmetric encryption or public keys may also make use of a public key infrastructure including a registration and certification authority, such as e.g. a hearing device manufacture or an authority associated with the hearing device manufacture. Participating parties, such as the two or more trusted parties, on the one hand, and/or one or more third parties interested in the data to be shared, on the other hand, may deposit their public keys at the registration authority. From these, for example, certificates signed with a private key of the certification authority may be generated and offered for download over the Internet.

According to an embodiment of the present technology, storing a definition of the respective trusted party comprises storing its public key in the hearing system; and generating the respective sharing approval by the device of this trusted party comprises generating and signing a sharing permission with a private key of this trusted party and, for example, transmitting the signed sharing permission to a predetermined receiving device of the hearing system for a next step of the present method.

According to an embodiment of the present technology, storing a definition of the respective trusted party comprises storing its voiceprint in the hearing system; and generating the respective sharing approval by the device of this trusted party comprises transmitting a predetermined sharing approval phrase and/or keyword read and/or uttered by this trusted party as an audio signal from its device to a predetermined receiving device of the hearing system and verifying the received audio signal by comparing it with the saved voiceprint of the respective trusted party.

According to an embodiment of the present technology, storing a definition of the respective trusted party comprises storing its symmetric key in the hearing system; and providing the sharing request to the device of the respective trusted party comprises encrypting a sharing request with the symmetric key of this trusted party and transmitting thus encrypted sharing request to its device. In this embodiment, the generation of the respective sharing approval by the device of this trusted party comprises generating and encrypting a sharing approval with the symmetric key of this trusted party and, for example, transmitting thus encrypted sharing approval to a predetermined receiving device of the hearing system for a next step of the present method. By using symmetric keys instead of asymmetric keys, memory space and/or calculation capacity of the hearing system may be spared.

According to an embodiment of the present technology, the method further comprises: storing connection data (e.g. a URL) for connecting to a predetermined central server as well as a public key of this central server in the hearing system. In this embodiment, storing a definition of the respective trusted party comprises storing an identification number (ID) and/or a public key of the respective trusted party in the central server; and providing the sharing request to the device of the respective trusted party comprises transmitting the sharing request from the central server to the device of the respective trusted party. This may, for instance, include providing a sharing request to said central server, whereupon it transmits the sharing request to the device of the respective trusted party. Further, in this embodiment, generating the respective sharing approval may comprise transmitting a signed sharing permission signed with a private key of the respective trusted party from its device to the central server.

With this embodiment, all authorizations may be centrally managed by the central server. Here, instead of storing public keys of trusted parties in the hearing device, for example, only an ID of the respective trusted party (e.g. the first or second trusted party) and the public key and the URL of the central server may be saved in the hearing device or elsewhere in the hearing system. Using this ID, the public key of the respective trusted party can be retrieved from the central server. The respective public key may, for example, be signed with a private key of the central server. The ID may comprise, by way of example only, one or more of the following: a random number; a phone number; a name; a customer number; a social insurance number of the respective party.

According to an embodiment of the present technology, the method further comprises: encrypting the data to be shared with a public key or with a symmetric key of the third party, prior to sharing the data. Thereby, the security of the method and system may be further increased.

According to an embodiment of the present technology, the step of generating the first and/or second sharing approval further comprises obtaining and checking a predefined context information comprising one or more of the following: data regarding the third party (in particular the name and profession of the third party); the kind of data to be shared; the time when the data was collected; the time when the sharing was requested (e.g. by the hearing device user or by the third party, such as an emergency service), i. e. the time the sharing request was generated or provided to the trusted parties; a time when the sharing can be done the earliest; a time when the sharing can be done the latest; a location of the hearing device from which the sharing is allowed, e.g. at home; a number and/or kind of trusted parties; a required kind of biometric data; and/or a required kind of out-of band response. In this embodiment, as the case may be, data regarding the third party and/or a further party may be digitally signed, too.

According to an embodiment of the present technology, the hearing system is configured such that the step of storing a definition of the respective trusted party is restricted to be performed only by a predetermined defining authority, whose connection data (e.g. a URL) and/or public key are saved in the hearing system. The defining authority may, for instance, be a superordinate defining authority, such as the manufacturer of the hearing device. It may also be that the superordinate defining authority may set an intermediary defining authority, e.g. a hearing professional. This may be done during fitting of the hearing device. In this case, the superordinate defining authority passes its authorization to save a definition of the trusted parties to the intermediary defining authority, e.g. by issuing a signed authorization, which may be signed with a private key of the superordinate defining authority and comprise the public kea of the intermediary defining authority. In the hearing device or system, a validation of this authorization may be implemented, e.g. by proving the signature of the authorization using the public key of the superordinate defining authority saved in the hearing system. It also may be that the authorization as the defining authority is provided with a time limit of validity. It may also be that two or more independent defining authorities are provided, e.g. such that at least two different trusted parties can only be defined by at least two different defining authorities. For example, a hearing care professional and his costumer do this together.

According to an embodiment of the present technology, the hearing system is configured such that the steps of storing a definition of the respective trusted party and/or of generating the respective sharing approval are at least partly restricted to a predetermined out-of-band-communication channel (such as only via a microphone of the hearing device by using a voice recognition software and/or acoustically encoded data such as DTMF, dual-tone multi-frequency, or only via predetermined wired or wireless communication channels, e.g. a wired interface, Bluetooth, WLAN, a conventional telephone service, landline, cellular, GSM (3G, 4G, 5G), SMS Service, e-Mail and/or the Internet), which is different from a data communication channel used for sharing the data saved in the hearing system with the third party device.

Further aspects of the present technology relate to a computer program for a protected sharing of data saved in a hearing system, which system includes at least a hearing device used by a hearing device user, with a third party device, which program, when being executed by a processor, is adapted to carry out the steps of the method as described above and in the following as well as to a computer-readable medium, in which such a computer program is stored. The computer program may have several parts running in different device, i.e. may be executed in a distributed environment.

For example, the computer program may be executed in a processor of a hearing device, which hearing device, for example, may be carried by a person, i.e. the user, behind the ear. The computer-readable medium may be a memory of this hearing device. The computer program also may be executed by a processor of a connected user device, such as a smartphone or any other type of mobile device, which may be a part of the hearing system, and the computer-readable medium may be a memory of the mobile device. It also may be that some steps of the method are performed by the hearing device and other steps of the method are performed by the mobile device. In general, some of the steps may be performed in a first device and other steps may be performed in a second device.

In general, a computer-readable medium may be a floppy disk, a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g. the Internet, which allows downloading a program code. The computer-readable medium may be a non-transitory or transitory medium.

A further aspect of the present technology relates to a hearing system comprising at least one hearing device, a device of a first trusted party and a device of a second trusted party, wherein the hearing system is adapted for performing the method described herein above and below. Apart from the hearing device mentioned above, the hearing system may further include, by way of example, a second hearing device used by the same user and/or a connected user device, such as a smartphone or other mobile device or personal computer, used by the same user and/or a central server connected with the hearing device in a predefined way over a data communication network, e.g. the Internet.

According to an embodiment of the present technology, the hearing device comprises: a microphone; a processor for processing a signal from the microphone; an output device for outputting the processed signal to an ear of the hearing device user; and a transceiver for exchanging data with the device of the first and/or second trusted party and/or with a connected user device and/or with a central server and/or with another hearing device.

The definition of the two or more trusted parties of the present method may be, for example, saved in the hearing device by programming. As described in more detail above, the programming may be performed e.g. by storing a public key of the respective trusted party in the hearing device. As also described above, the public key may be a part of a certificate of the respective party. Furthermore, a time limit may be provided for the validity of the respective public key or certificate.

The definition of the two or more trusted parties may, for instance, be performed during a first fitting session, e.g. in an office of a hearing professional, where the at least one hearing device is being fitted to its user. In the course of this, the hearing device may, for instance, be connected with a fitting computer (serving as an intermediary defining authority), e.g. by wireless communication such as Bluetooth. The fitting computer, in its turn, may be connected over a data communication network such as the Internet with a superordinate defining authority, such as the manufacturer of the hearing device.

It may also be implemented that the hearing device user may change the definition of the trusted parties without visiting the office of the hearing professional again. To this end, for example, the public key of the hearing professional or another defining authority may be saved in the hearing device or system. This may be in the context of a so called "remote fitting" where an audiologist can remotely program a hearing aid (tele audiology). It may also be in the context of a self-fitting where the hearing aid user adjusts the hearing aid by him- or herself.

Using the public key of the defining authority, the user may effect a redefinition of trusted parties in a verified manner, in that the corresponding programming commands transmitted from the defining authority to the hearing system are signed with the private key of the defining authority.

Depending on the security requirements, the present technology, e.g. the steps of the method described herein, may be primarily implemented in the hearing device or, alternatively, in a mobile application running on another device of the hearing system, e.g. on a smartphone (via a corresponding Smartphone App) or another connected user device. The more of the steps and aspects of the present technology are implemented in the hearing device, the higher level of protection for the data sharing against unauthorized hacking might be achievable. To this end, in particular, a so-called Trusted Platform Module may be a part of the hearing device, e.g. such as disclosed in EP 3 236 674 A1 as described at the outset. By a Trusted Platform Module, the hearing device may be, for instance, provided with public key security functions. That is, a Trusted Platform Module may include a secure crypto processor, a random generator, as well as a protected memory for private keys, which is only readable by the cryptoprocessor, but not by the main processor. The public key of the device, the public key of a hearing device manufacturer and an operating system may be saved in a storage region of a non-volatile memory of the hearing device which is protected against modification, i.e. in a sealed storage region.

It has to be understood that features of the method as described above and in the following may be features of the computer program, the computer-readable medium and the hearing system as described above and in the following, and vice versa.

These and other aspects of the present technology will be apparent from and elucidated with reference to the embodiments described hereinafter.

### BRIEF DESCRIPTION OF THE DRAWINGS

Below, embodiments of the present technology are described in more detail with reference to the attached drawings.
Fig. 1 schematically shows a block diagram of a hearing system according to an embodiment of the present technology.
Fig. 2 shows a flow diagram for a method according to an embodiment of the present technology for a protected sharing of data saved in the hearing system of Fig. 1 with a third party.
Fig. 3 schematically shows a sequence diagram visualizing the method of Fig. 2 according to a further embodiment of the present technology involving cascaded encryption.
Fig. 4 schematically shows a sequence diagram visualizing the method of Fig. 2 according to a further embodiment of the present technology involving signed sharing permissions.
Fig. 5 schematically shows a sequence diagram visualizing the method of Fig. 2 according to a further embodiment of the present technology involving voiceprints of trusted parties.
Fig. 6 schematically shows a sequence diagram visualizing the method of Fig. 2 according to a further embodiment of the present technology involving a central server managing the sharing approvals.

The reference symbols used in the drawings, and their meanings, are listed in summary form in the list of reference symbols. In principle, identical parts are provided with the same reference symbols in the figures.

### DETAILED DESCRIPTION OF EXEMPLARY EMBODIMENTS

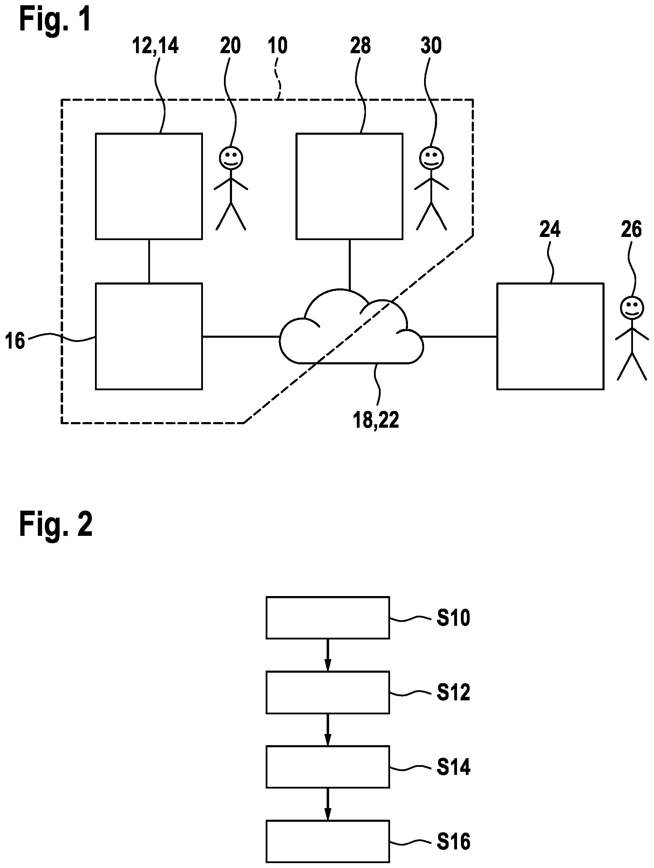

Fig. 1 shows a hearing system 10 which comprises two hearing devices 12, 14 and a connected user device 16.

Each of the two hearing devices 12, 14 may be worn by a user 20 behind the ear and/or in the ear. For example, the hearing device 12 may be a left hearing device for the left ear and the hearing device 14 may be a right hearing device for the right ear. The two hearing devices 12, 14 may constitute a binaural hearing system. The hearing devices 12, 14 may be hearing aids adapted for compensating a hearing loss of the user 20. At least one of the hearing devices 12, 14 may comprise one or more sensors, e.g. accelerometers, pulse oximetry or electroencephalography sensors, collecting health data of the user 20. The two hearing devices 12, 14 may be adapted for exchanging data wirelessly, for example via Bluetooth (e.g. BLE, Low Energy). For example, it may be that an audio signal received and/or processed by the first hearing device 12 and/or sensor data collected by its sensors may be transmitted to the second hearing device 14, and vice versa.

The connected user device 16, which may be a tablet computer or smartphone, also may be adapted for exchanging data wirelessly with one or both of the hearing devices 12, 14, for example via Bluetooth. Furthermore, the hearing devices 12, 14 and/or the user device 16 may be adapted for exchanging data with one or more data communication networks 18, such as the Internet and/or a telephone network. Via the data communication networks 18, the hearing devices 12, 14 and/or the user device 16 may also be able to connect to a predetermined remote central server 22, which may also be regarded as a part of the hearing system 10, as schematically indicated by a dashed line in Fig. 1.

The hearing system 10 is adapted for performing a method according to the present technology for a protected sharing of data saved in the hearing system 10, e.g. health data of the user 20 saved in the hearing devices 12 and 14, with a device 24, such as a personal computer, of a third party 26, e.g. a health professional or an emergency service, who might need this data to help the user 20 in a health treatment or an emergency case. To this end, the hearing system 10 further comprises a device 28, e.g. a mobile device such as a smartphone, of a significant other 30, who may, for example, be a spouse or another family member of the user 20.

Fig. 2 shows a flow diagram for a method for a protected sharing of data saved in the hearing system 10 with the third party device 24.

A first step S10 of the method may be performed, for instance, during a fitting session in an office of a hearing professional, where the hearing devices 12 and 14 are fitted to their user 20. In step S10, storing a definition of a first trusted party and of a second trusted party in the hearing system 10 is performed. Beside storing such a definition in a hearing device 12 or 14, this may also include storing the definition in the connected user device 16 and/or in the central server 22. In order to save the definition of the trusted parties, the hearing device 12 and/or 14 may, for instance, be connected with a fitting computer (serving as an intermediary defining authority), e.g. by wireless communication such as Bluetooth.

In the present example, the first trusted party is the hearing device user 20, whereas the second trusted party is a significant other 30, such as his/her spouse.

In step S12, a sharing request for the data to be shared is provided to the device 16 of the first trusted party (in this example to the smartphone of the hearing device user 20) and to the device 28 of the second trusted party (in this example to a smartphone or personal computer of the significant other 30). The sharing request may, for instance, be generated by the user 20 himself, using his smartphone or one of the hearing devices 12 or 14. Alternatively, e.g. in an emergency case, the sharing request may also be generated by the third party 26, such as an emergency service.

In step S14, a first sharing approval is generated by the device 16 of the first trusted party and a second sharing approval is generated by the device 28 of the second trusted party.

In final step S16, sharing of the data with the third party device 24 is only performed, if both sharing approvals have been successfully generated.

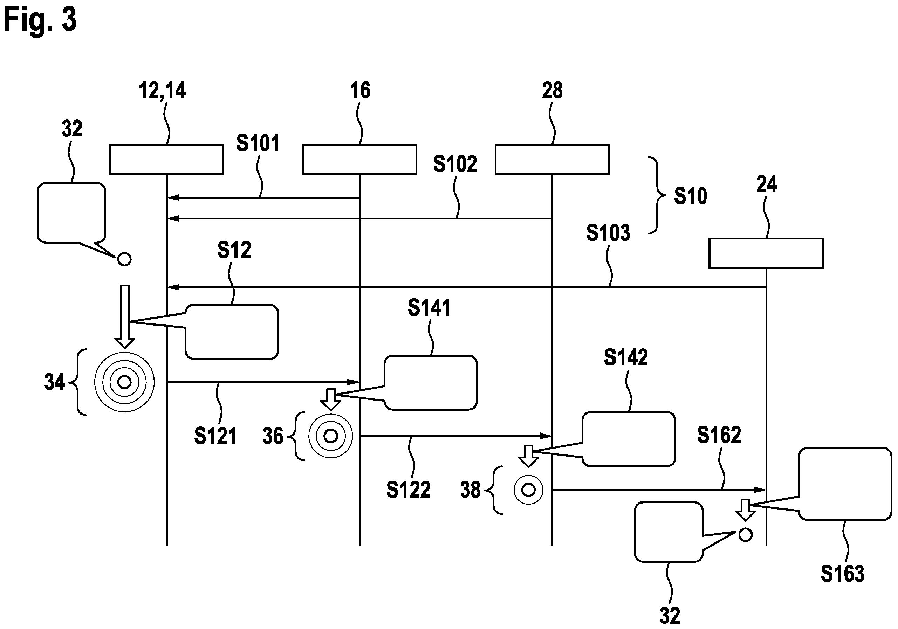

A number of possible implementations of the steps S10 to S16 will be visualized in the following with reference to Fig. 3 to 6.

Fig. 3 schematically shows a block diagram visualizing the method of Fig. 2 according to a first embodiment of the present technology, involving cascaded encryption. In this embodiment, storing a definition of the respective trusted party in step S10 comprises storing S101, S102 its public key in the hearing device 12 or 14. Furthermore, providing the sharing request to the device 16, 28 of the respective trusted party in step S12 comprises encrypting plain sensor data 32 collected and saved in the hearing device 12 or 14 with the public key of the respective trusted party and transmitting S121, S122 thus encrypted data 34 to its respective device 16, 28. Further, the generation of the respective sharing approval by the device 16, 28 of the respective trusted party in step S14 comprises decrypting S141, S142 the data with a private key of the respective trusted party and transmitting thus decrypted data 36, 38 to a predetermined receiving device of the hearing system 10 for a next step of the present method.

In other words, as illustrated in Fig. 3, a cascaded encryption may be implemented by this embodiment. That is, plain sensor data 32, which is collected and saved in the hearing device 12 or 14 and is to be shared with the third party 26, is encrypted in step S12 with the public key of the first trusted party, with the public key of the second trusted party and, optionally, also with a public key of the third party 26 previously obtained in an additional step S103. Thus encrypted data 34 is transmitted in substep S121 to the device 16 of the first trusted party, who can prove all the necessary details and approve the sharing by decrypting S141 the data 34 in its device 16 with its private key and by transmitting S122 thus decrypted data 36 to the device 28 of the second trusted party. The second trusted party can proceed in the same manner and transmit S162 the data 38 decrypted S142 with its private key in its device 28 to the device 24 of the third party 26, thereby sharing this data with the third party 26. In this example, the shared data 38 is, finally, decrypted S163 by the third party 26 with its private key in its device 24, which renders plain sensor data 32, whereby the sharing step S16 is completed.

The method illustrated in Fig. 3 may, in particular, comprise the following steps:
- Storing a first public key of a first trusted party in the hearing device 12, 14
- Storing a second public key of a second trusted party in the hearing device 12, 14
- Acquiring sensor data with the sensors
- Analyzing the sensor data in the hearing device 12, 14
- Reporting a health condition to the hearing device user 20
- Selecting a third party 26 (e.g. medical professional) by the hearing device user 20
- Generating by the connected user device 16 a request to share the data with the third party (e.g. using a suitable app running on the device 16); the hearing device 12, 14 receiving this sharing request
- Obtaining a public key of the third party 26 from the third party device 24
- Encrypting the data in the hearing device 12, 14 with the public key of the third party 26
- Encrypting the data in the hearing device 12, 14 with the first public key
- Encrypting the data in the hearing of the third party 26 with the second public key
- Transmitting the data from the hearing device to the device 16 of the first trusted party
- Decrypting the data by the first trusted party in its device 16
- Transmitting the data from device 16 of the first trusted party to the device 28 of the second trusted party
- Decrypting the data by the second trusted party in its device 28
- Transmitting the data from the device 28 of the second trusted party to the device 24 of the third party 26
- Decrypting the shared data by the third party 26 in its device 24.

Encrypting the data in the hearing device 12, 14 with the first public key and encrypting the data in the hearing of the third party 26 with the second public key sets the order of decryption. However it is also possible to generate two sets of data, which are encrypted with the first public key and with the second pubic key in different orders.

Furthermore, the data may comprise meta information, which medical data are encoded into the data, who should receive the data. The meta information may be added to the data after the first encrypting step, such that the corresponding trusted party is able to read the meta information after the first decryption step without the need for performing the second decryption step.

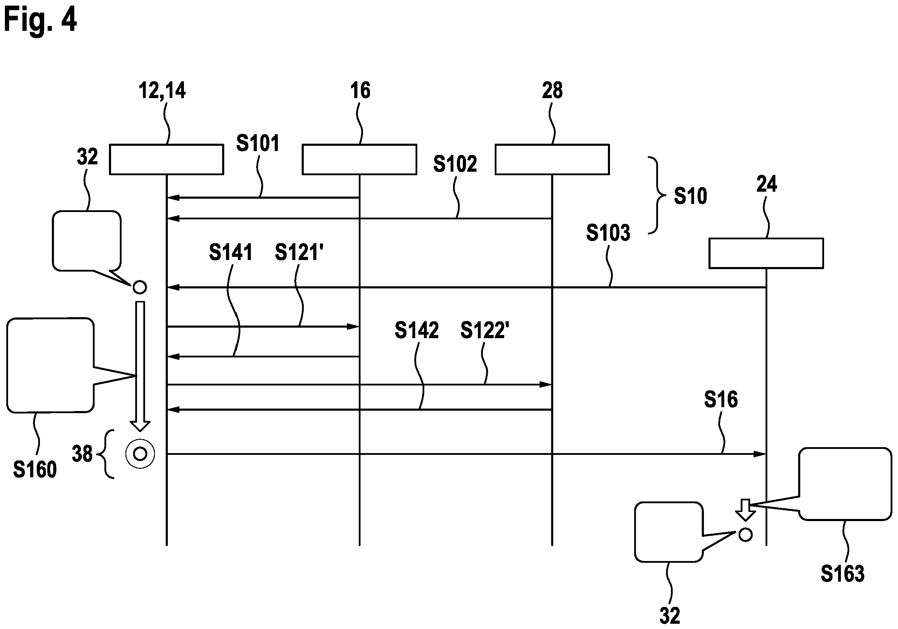

Fig. 4 schematically shows a block diagram visualizing the method of Fig. 2 according to a second embodiment of the present technology involving signed sharing permissions. In this embodiment, storing a definition of the respective trusted party in step S10 comprises storing S101, S102 its public key in the hearing device 12 or 14; and providing the sharing request to the device 16, 28 of the respective trusted party in step S12 simply comprises transmitting S121', S122' the sharing request to its respective device 16, 28 from the hearing device 12, 14. Further, the generation of the respective sharing approval by the device 16, 28 of the respective trusted party in step S14 comprises generating a sharing permission signed with a private key of this trusted party and transmitting S141, S142 the signed sharing permission to a predetermined receiving device of the hearing system 10, such as to the hearing device 12 or 14, for a next step of the present method, i.e. for the step S16 of sharing the data. Similarly to Fig. 3, prior to be shared, the plain sensor data 32 is encrypted in step S160 with a public key of the third party 26 obtained from its device 24 in step S103. The shared encrypted data 38 may then be decrypted by the third party 26 in its device 24 in step S163, so as to obtain plain sensor data 32 and/or data derived from it, e.g. diagnostic data, and, thus, complete the sharing.

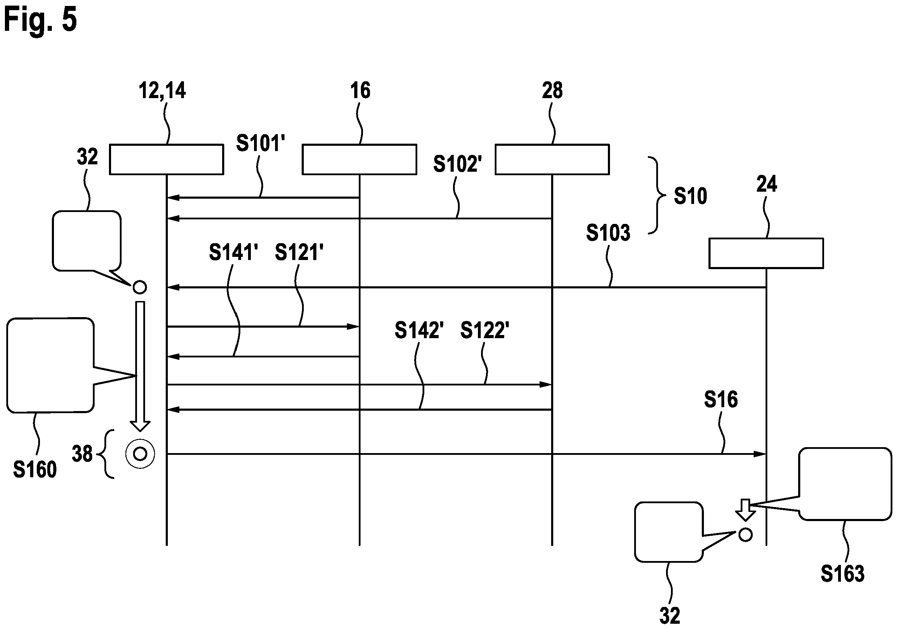

Fig. 5 schematically shows a block diagram visualizing the method of Fig. 2 according to a third embodiment of the present technology involving voiceprints of trusted parties. This embodiment only differs from the one shown in Fig. 4 in that storing a definition of the respective trusted party in step S10 comprises storing its voiceprint S101', S102' in the hearing device 12 or 14; and in that the generation of the respective sharing approval by the device 16, 28 of the respective trusted party in step S14 comprises transmitting S141', S142' a predetermined sharing approval phrase read by this trusted party as an audio signal from its device 16, 28 to the hearing device 12 or 14 and verifying the received audio signal by comparing it with the saved voiceprint of the respective trusted party. It also may be that instead of the complete audio signal solely features of the audio signal are transmitted. The analysis of the audio signal may be performed in a server. The audio signal may be transmitted "out of band", for example via the hearing device microphone and/or via the classical telephone network. Alternatively, or in addition to a voice print there may be other biometric methods, such as iris scan and/or fingerprint.

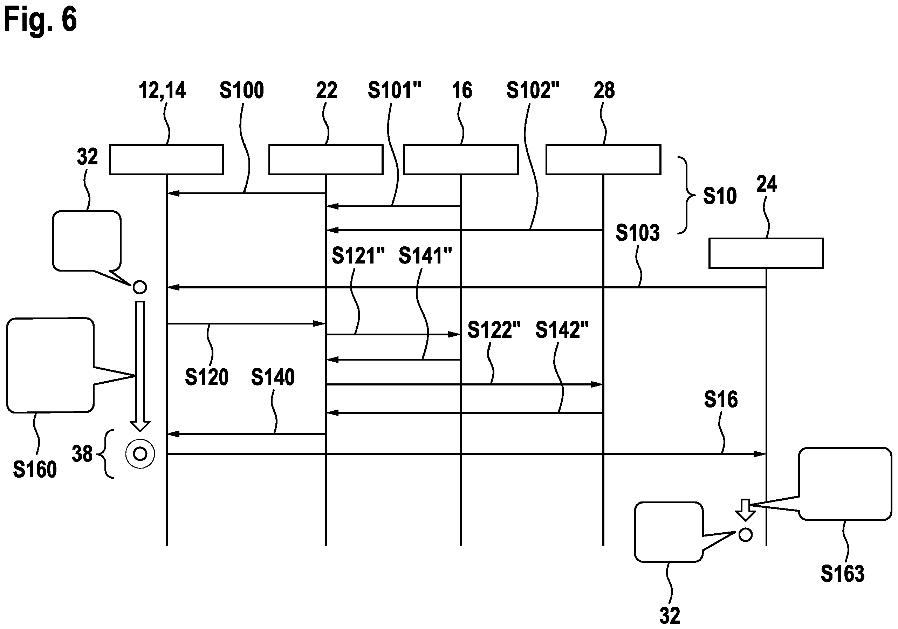

Fig. 6 schematically shows a block diagram visualizing the method of Fig. 2 according to a fourth embodiment of the present technology involving a central server managing the sharing approvals. According to this embodiment of the present technology, the method further comprises a step S100 of storing connection data (e.g. an ID and/or a URL) for connecting to a predetermined central server 22 as well as a public key of this central server 22 in the hearing system 10, e.g. in the hearing device 12 or 14.

Further, in Fig. 6, storing a definition of the respective trusted party in step S10 comprises storing S101", S102"an ID and/or a public key of the respective trusted party in the central server 22. Further, providing the sharing request to the device 16, 28 of the respective trusted party in step S12 comprises providing S120 a sharing request to the central server 22, whereupon it transmits S121", S122" the sharing request to the device 16, 28 of the respective trusted party. Further, in this embodiment, the step S14 of generating the respective sharing approval comprises transmitting S141", S142" a signed sharing permission signed with a private key of the respective trusted party from its device 16, 28 to the central server 22, whereupon the central server 22 transmits S140 a signed sharing permission signed with a private key of the central server 22 to the hearing device 12 or 14. Similarly to Figs. 4 and 5, prior to be shared, the plain sensor data 32 is encrypted in step S160 with a public key of the third party 26 obtained from its device 24 in step S103. The shared data 38 may then also be decrypted by the third party 26 in its device 24 in step S163, so as to obtain plain sensor data 32 and, thus, complete the sharing.

With this embodiment, all authorizations may be centrally managed by the central server. Here, instead of storing public keys of trusted parties in the hearing device, for example, only an ID of the respective trusted party (e.g. the first or second trusted party) and the public key and the URL of the central server may be saved in the hearing device 12, 14 or in the central server 22 of the hearing system 10. Using this ID, the public key of the respective trusted party can be retrieved from the central server 22. The respective public key may, for example, be signed with a private key of the central server. The ID may comprise, by way of example only, one or more of the following: a random number; a phone number; a name; a customer number; a social insurance number.

The examples of Fig. 3 to 6 apply asymmetric encryption, private and public keys likewise. They may also be embodied fully or partially based on symmetric encryption.

While the present technology has been illustrated and described in detail in the drawings and foregoing description, such illustration and description are to be considered illustrative or exemplary and not restrictive; the present technology is not limited to the disclosed embodiments. Other variations to the disclosed embodiments can be understood and effected by those skilled in the art and practicing the claimed present technology, from a study of the drawings, the disclosure, and the appended claims. In the claims, the word "comprising" does not exclude other elements or steps, and the indefinite article "a" or "an" does not exclude a plurality. A single processor or controller or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SYMBOLS

- 10: hearing system
- 12: first hearing device
- 14: second hearing device
- 16: connected user device, device of a first trusted party
- 18: data communication network(s)
- 20: hearing device user, first trusted party
- 22: central server
- 24: device of a third party
- 26: third party, with whom data is to be shared
- 28: device of a second trusted party
- 30: significant other, second trusted party
- 32: plain sensor data
- 34: sensor data encrypted with the public keys of the first and the second trusted party, as well as with the public key of the third party
- 36: sensor data only encrypted with the public keys of the second trusted party and of the third party
- 38: sensor data only encrypted with the public key of the third party
- S10: storing a definition of a first trusted party and of a second trusted party in the hearing system
- S12: providing a sharing request to the device of the first trusted party and to the device of the second trusted party
- S14: generating a first sharing approval by the device of the first trusted party and a second sharing approval by the device of the second trusted party
- S16: sharing of the data with the third party device
- 5101: storing a public key of the first trusted party in the hearing device
- 5102: storing a public key of the second trusted party in the hearing device
- S101': storing a voiceprint of the first trusted party in the hearing device
- S102': storing a voiceprint of the second trusted party in the hearing device
- S101": storing an ID and/or a public key of the first trusted party in the central server
- S102": storing an ID and/or a public key of the second trusted party in the central server
- S100: storing connection data and/or a public key of the central server in the hearing system
- S103: storing a public key of the third party in the hearing device
- S120: providing a sharing request to the central server
- 5121': transmitting a sharing request to the device of the first trusted party from the hearing device
- 5122': transmitting a sharing request to the device of the second trusted party from the hearing device
- S121": transmitting a sharing request to the device of the first trusted party from the central server
- S122": transmitting a sharing request to the device of the second trusted party from the central server
- 5121: encrypting the data to be shared with the public key of the first trusted party and transmitting it, as a sharing request, to its device
- S122: encrypting the data to be shared with the public key of the second trusted party and transmitting it, as a sharing request, to its device
- S141: decrypting the data with the private key of the first trusted party, or transmitting a sharing permission signed therewith to the hearing device
- S142: decrypting the data with the private key of the second trusted party, or transmitting a sharing permission signed therewith to the hearing device
- S141': transmitting a predetermined sharing approval phrase read by the first trusted party as an audio signal from its device and verifying the received audio signal by comparing it with the saved voiceprint
- S142': transmitting a predetermined sharing approval phrase read by the second trusted party as an audio signal from its device and verifying the received audio signal by comparing it with the saved voiceprint
- S141": transmitting a sharing permission signed with the private key of the first trusted party to the central server
- S142": transmitting a sharing permission signed with the private key of the second trusted party to the central server
- S140: transmitting a signed sharing permission signed with a private key of the central server to the hearing device
- S160: encrypting data to be shared with a public key of the third party
- S163: decrypting the shared data with a private key of the third party in its device

## Claims

1. A method for a protected sharing of data stored in a hearing system (10), which comprises at least a hearing device (12, 14) used by a hearing device user (20), with a third party device (24), the method comprising:
storing a definition of a first trusted party and of a second trusted party in the hearing system (10);
providing a sharing request for the data to be shared to a device (16) of the first trusted party and to a device (28) of the second trusted party;
generating a first sharing approval by the device (16) of the first trusted party and a second sharing approval by the device (28) of the second trusted party, and sharing the data with the third party device (24) upon generation of the first and the second sharing approval.

2. The method of claim 1,
wherein the data (32) to be shared is collected by a sensor and/or is derived from data collected by a sensor, which is a part of the hearing device (12, 14).

3. The method of claim 1 or 2, further comprising:
storing a definition of at least one further trusted party in the hearing system (10);
providing a sharing request to a device of each further trusted party; and
generating a further sharing approval by the device of each further trusted party, and sharing the data with the third party device (24) upon generation of the first sharing approval, the second sharing approval, and the at least one further sharing approval.

4. The method of one of the previous claims, wherein
storing a definition of the respective trusted party comprises storing its public key in the hearing system (10);
providing the sharing request to the device (16, 28) of the respective trusted party comprises encrypting the data to be shared with the public key of the respective trusted party and transmitting the encrypted data to its device (16, 28); and
generating the respective sharing approval comprises decrypting the data to be shared with a private key of the respective trusted party in its device (16, 28).

5. The method of one of the previous claims, wherein
storing a definition of the respective trusted party comprises storing its public key in the hearing system (10); and
generating the respective sharing approval comprises signing a sharing permission with a private key of the respective trusted party in its device (16, 28).

6. The method of one of the previous claims, wherein
storing a definition of the respective trusted party comprises storing its voiceprint in the hearing system (10); and
generating the respective sharing approval comprises transmitting a predetermined sharing approval phrase and/or keyword, read and/or uttered by the respective trusted party as an audio signal from the device (16, 28) of the respective trusted party to a predetermined receiving device of the hearing system (10) and verifying the received audio signal by comparing it with the voiceprint of this trusted party.

7. The method of one of the previous claims, wherein
storing a definition of the respective trusted party comprises storing its symmetric key in the hearing system (10);
providing the sharing request to the device (16, 28) of the respective trusted party comprises encrypting a sharing request with the symmetric key of this trusted party and transmitting thus encrypted sharing request to its device (16, 28); and
generating the respective sharing approval comprises encrypting a sharing approval with the symmetric key of the respective trusted party in its device (16, 28).

8. The method of one of the previous claims, wherein
connection data for connecting to a predetermined central server (22) as well as its public key are saved in the hearing system (10);
storing a definition of the respective trusted party comprises storing an identification number and/or a public key of the respective trusted party in said central server (22);
providing the sharing request to the device (16, 28) of the respective trusted party comprises transmitting the sharing request from said central server (22) to the device (16, 28) of the respective trusted party; and
generating the respective sharing approval comprises transmitting a signed sharing permission signed with a private key of the respective trusted party from its device (16, 28) to said central server (22).

9. The method of one of the previous claims,
wherein, prior to be shared, the data is encrypted with a public key of the third party (26) or with a symmetric key of the third party (26).

10. The method of one of the previous claims, wherein the step of generating the first and/or second sharing approval comprises obtaining and checking a predefined context information comprising one or more of the following:
data regarding the third party (26);
a kind of data to be shared;
a time when the data was collected;
a time when the sharing request was generated;
a time when the sharing can be done the earliest;
a time when the sharing can be done the latest;
a location of the hearing device from which the sharing is allowed, e.g. at home;
a number and/or kind of trusted parties;
a required kind of biometric data;
a required kind of out-of band response.

11. The method of one of the previous claims,
wherein the hearing system (10) is configured such that the step of storing a definition of the respective trusted party is restricted to be performed only by a predetermined defining authority, whose connection data and/or public key are saved in the hearing system (10).

12. The method of one of the previous claims,
wherein the hearing system (10) is configured such that the steps of storing a definition of the respective trusted party and/or of generating the respective sharing approval are at least partly restricted to a predetermined out-of-band-communication channel, which is different from a data communication channel used for sharing the data with the third party device (24).

13. A computer program for a protected sharing of data saved in a hearing system (10), which system comprises at least a hearing device (12, 14) used by a hearing device user (20), with a third party device (24), which program, when being executed by a processor, is adapted to carry out the steps of the method of one of the previous claims.

14. A computer-readable medium, in which a computer program according to claim 13 is stored.

15. A hearing system (10) comprising a hearing device (12, 14), a device (16) of a first trusted party and a device (28) of a second trusted party, wherein the hearing device (12, 14) comprises:
a microphone;
a processor for processing a signal from the microphone;
an output device for outputting the processed signal to an ear of a hearing device user;
a transceiver for exchanging data with the device of the first and/or second trusted party and/or with a connected user device and/or with a central server and/or with another hearing device,
wherein the hearing system (10) is adapted for performing the method of one of claims 1 to 12.

## Patentansprüche

1. Verfahren zur geschützten gemeinsamen Nutzung von Daten, die in einem Hörsystem (10) gespeichert sind, das mindestens ein Hörgerät (12, 14) umfasst, das durch einen Hörgerätbenutzer (20) verwendet wird, mit einem Fremdgerät (24), wobei das Verfahren umfasst:
Speichern einer Definition einer ersten vertrauenswürdigen Partei und einer zweiten vertrauenswürdigen Partei in dem Hörsystem (10);
Übermitteln einer Freigabeanforderung für die gemeinsam zu nutzenden Daten an ein Gerät (16) der ersten vertrauenswürdigen Partei und an ein Gerät (28) der zweiten vertrauenswürdigen Partei;
Generieren einer ersten Freigabegenehmigung durch das Gerät (16) der ersten vertrauenswürdigen Partei und einer zweiten Freigabegenehmigung durch das Gerät (28) der zweiten vertrauenswürdigen Partei, und gemeinsames Nutzen der Daten mit dem Fremdgerät (24) nach dem Generieren der ersten und der zweiten Freigabegenehmigung.

2. Verfahren nach Anspruch 1,
wobei die gemeinsam zu nutzenden Daten (32) durch einen Sensor gesammelt werden und/oder von Daten abgeleitet werden, die durch einen Sensor gesammelt werden, der Teil des Hörgeräts (12, 14) ist.

3. Verfahren nach Anspruch 1 oder 2, des Weiteren umfassend:
Speichern einer Definition mindestens einer weiteren vertrauenswürdigen Partei in dem Hörsystem (10);
Übermitteln einer Freigabeanforderung an ein Gerät jeder weiteren vertrauenswürdigen Partei; und
Generieren einer weiteren Freigabegenehmigung durch das Gerät jeder weiteren vertrauenswürdigen Partei und gemeinsames Nutzen der Daten mit dem Fremdgerät (24) nach dem Generieren der ersten Freigabegenehmigung, der zweiten Freigabegenehmigung und der mindestens einen weiteren Freigabegenehmigung.

4. Verfahren nach einem der vorangehenden Ansprüche, wobei
das Speichern einer Definition der jeweiligen vertrauenswürdigen Partei das Speichern ihres öffentlichen Schlüssels in dem Hörsystem (10) umfasst;
das Übermitteln der Freigabeanforderung an das Gerät (16, 28) der jeweiligen vertrauenswürdigen Partei das Verschlüsseln der gemeinsam zu nutzenden Daten mit dem öffentlichen Schlüssel der jeweiligen vertrauenswürdigen Partei und das Senden der verschlüsselten Daten an ihr Gerät (16, 28) umfasst; und
das Generieren der jeweiligen Freigabegenehmigung das Entschlüsseln der gemeinsam zu nutzenden Daten mit einem privaten Schlüssel der jeweiligen vertrauenswürdigen Partei in ihrem Gerät (16, 28) umfasst.

5. Verfahren nach einem der vorangehenden Ansprüche, wobei
das Speichern einer Definition der jeweiligen vertrauenswürdigen Partei das Speichern ihres öffentlichen Schlüssels in dem Hörsystem (10) umfasst; und
das Generieren der jeweiligen Freigabegenehmigung das Signieren einer Freigabeerlaubnis mit einem privaten Schlüssel der jeweiligen vertrauenswürdigen Partei in ihrem Gerät (16, 28) umfasst.

6. Verfahren nach einem der vorangehenden Ansprüche, wobei
das Speichern einer Definition der jeweiligen vertrauenswürdigen Partei das Speichern ihres Stimmabdrucks in dem Hörsystem (10) umfasst; und
das Generieren der jeweiligen Freigabegenehmigung umfasst: Senden einer zuvor festgelegten Freigabegenehmigungsphrase und/oder eines zuvor festgelegten Freigabegenehmigungsschlüsselwortes, die bzw. das durch die jeweilige vertrauenswürdige Partei gelesen und/oder geäußert wird, als ein Audiosignal von dem Gerät (16, 28) der jeweiligen vertrauenswürdigen Partei an ein zuvor festgelegtes Empfangsgerät des Hörsystems (10) und Verifizieren des empfangenen Audiosignals durch Vergleichen des empfangenen Audiosignals mit dem Stimmabdruck dieser vertrauenswürdigen Partei.

7. Verfahren nach einem der vorangehenden Ansprüche, wobei
das Speichern einer Definition der jeweiligen vertrauenswürdigen Partei das Speichern ihres symmetrischen Schlüssels in dem Hörsystem (10) umfasst;
das Übermitteln der Freigabeanforderung an das Gerät (16, 28) der jeweiligen vertrauenswürdigen Partei das Verschlüsseln einer Freigabeanforderung mit dem symmetrischen Schlüssel dieser vertrauenswürdigen Partei und das Senden der so verschlüsselten Freigabeanforderung an ihr Gerät (16, 28) umfasst; und
das Generieren der jeweiligen Freigabegenehmigung das Verschlüsseln einer Freigabegenehmigung mit dem symmetrischen Schlüssel der jeweiligen vertrauenswürdigen Partei in ihrem Gerät (16, 28) umfasst.

8. Verfahren nach einem der vorangehenden Ansprüche, wobei Verbindungsdaten zum Verbinden mit einem zuvor festgelegten zentralen Server (22) sowie dessen öffentlicher Schlüssel in dem Hörsystem (10) gespeichert werden;
das Speichern einer Definition der jeweiligen vertrauenswürdigen Partei das Speichern einer Identifikationsnummer und/oder eines öffentlichen Schlüssels der jeweiligen vertrauenswürdigen Partei in dem zentralen Server (22) umfasst;
das Übermitteln der Freigabeanforderung an das Gerät (16, 28) der jeweiligen vertrauenswürdigen Partei das Senden der Freigabeanforderung von dem zentralen Server (22) an das Gerät (16, 28) der jeweiligen vertrauenswürdigen Partei umfasst; und
das Generieren der jeweiligen Freigabeerlaubnis das Senden einer signierten Freigabeerlaubnis, die mit einem privaten Schlüssel der jeweiligen vertrauenswürdigen Partei signiert ist, von ihrem Gerät (16, 28) an den zentralen Server (22) umfasst.

9. Verfahren nach einem der vorangehenden Ansprüche,
wobei die Daten, vor der gemeinsamen Nutzung, mit einem öffentlichen Schlüssel der fremden Person (26) oder mit einem symmetrischen Schlüssel der fremden Person (26) verschlüsselt werden.

10. Verfahren nach einem der vorangehenden Ansprüche, wobei der Schritt des Generierens der ersten und/oder der zweiten Freigabegenehmigung das Erhalten und Überprüfen einer vordefinierten Kontextinformation umfasst, die eines oder mehrere von Folgendem umfasst:
Daten über die fremde Person (26);
eine Art von gemeinsam zu nutzenden Daten;
einen Zeitpunkt, wann die Daten gesammelt wurden;
einen Zeitpunkt, wann die Freigabeanforderung generiert wurde;
einen Zeitpunkt, wann die gemeinsame Nutzung frühestens stattfinden kann;
einen Zeitpunkt, wann die gemeinsame Nutzung spätestens stattfinden kann;
einen Standort des Hörgerätes, von dem aus die gemeinsame Nutzung erlaubt ist, zum Beispiel zu Hause;
eine Anzahl und/oder Art von vertrauenswürdigen Parteien;
eine erforderliche Art von biometrischen Daten;
eine erforderliche Art von Außerband-Antwort.

11. Verfahren nach einem der vorangehenden Ansprüche,
wobei das Hörsystem (10) so eingerichtet ist, dass der Schritt des Speicherns einer Definition der jeweiligen vertrauenswürdigen Partei nur durch eine zuvor festgelegte Definitionsautorität durchgeführt werden kann, deren Verbindungsdaten und/oder öffentlicher Schlüssel in dem Hörsystem (10) gespeichert sind.

12. Verfahren nach einem der vorangehenden Ansprüche,
wobei das Hörsystem (10) so eingerichtet ist, dass die Schritte des Speicherns einer Definition der jeweiligen vertrauenswürdigen Partei und/oder des Generierens der jeweiligen Freigabegenehmigung mindestens teilweise auf einen zuvor festgelegten Außerband-Kommunikationskanal beschränkt sind, der sich von einem Datenkommunikationskanal unterscheidet, der für die gemeinsame Nutzung der Daten mit dem Fremdgerät (24) verwendet wird.

13. Computerprogramm für eine geschützte gemeinsame Nutzung von Daten, die in einem Hörsystem (10) gespeichert sind, wobei das System mindestens ein Hörgerät (12, 14) umfasst, das durch einen Hörgerätbenutzer (20) verwendet wird, mit einem Fremdgerät (24), wobei das Programm, wenn es durch einen Prozessor ausgeführt wird, dafür ausgelegt ist, die Schritte des Verfahrens nach einem der vorangehenden Ansprüche auszuführen.

14. Computerlesbares Medium, auf dem ein Computerprogramm nach Anspruch 13 gespeichert ist.

15. Hörsystem (10), umfassend ein Hörgerät (12, 14), ein Gerät (16) einer ersten vertrauenswürdigen Partei und ein Gerät (28) einer zweiten vertrauenswürdigen Partei, wobei das Hörgerät (12, 14) umfasst:
ein Mikrofon;
einen Prozessor zum Verarbeiten eines Signals von dem Mikrofon;
ein Ausgabegerät zum Ausgeben des verarbeiteten Signals an ein Ohr eines Hörgerätbenutzers;
einen Sender/Empfänger zum Austauschen von Daten mit dem Gerät der ersten und/oder zweiten vertrauenswürdigen Partei und/oder mit einem verbundenen Benutzergerät und/oder mit einem zentralen Server und/oder mit einem anderen Hörgerät, wobei das Hörsystem (10) dafür ausgelegt ist, das Verfahren nach einem der Ansprüche 1 bis 12 auszuführen.

## Revendications

1. Procédé de partage protégé de données stockées dans un système auditif (10), qui comprend au moins un appareil auditif (12, 14) utilisé par un utilisateur d'appareil auditif (20), avec un dispositif tiers (24), le procédé comprenant :
le stockage d'une définition d'une première partie de confiance et d'une seconde partie de confiance dans le système auditif (10) ;
la fourniture d'une demande de partage pour les données à partager à un dispositif (16) de la première partie de confiance et à un dispositif (28) de la seconde partie de confiance ;
la génération d'une première approbation de partage par le dispositif (16) de la première partie de confiance et d'une seconde approbation de partage par le dispositif (28) de la seconde partie de confiance, et le partage des données avec le dispositif tiers (24) lors de la génération de la première et de la seconde approbation de partage.

2. Procédé selon la revendication 1,
dans lequel les données (32) à partager sont collectées par un capteur et/ou sont dérivées de données collectées par un capteur faisant partie de l'appareil auditif (12, 14).

3. Procédé selon la revendication 1 ou 2, comprenant en outre :
le stockage d'une définition d'au moins une autre partie de confiance dans le système auditif (10) ;
la fourniture d'une demande de partage à un dispositif de chaque autre partie de confiance ; et
la génération d'une autre approbation de partage par le dispositif de chaque autre partie de confiance, et le partage des données avec le dispositif tiers (24) lors de la génération de la première approbation de partage, de la seconde approbation de partage, et de l'au moins une autre approbation de partage.

4. Procédé selon l'une des revendications précédentes, dans lequel
le stockage d'une définition de la partie de confiance respective comprend le stockage de sa clé publique dans le système auditif (10) ;
la fourniture de la demande de partage au dispositif (16, 28) de la partie de confiance respective comprend le chiffrement des données à partager avec la clé publique de la partie de confiance respective et la transmission des données chiffrées à son dispositif (16, 28) ; et
la génération de l'approbation de partage respective comprend le déchiffrement des données à partager avec une clé privée de la partie de confiance respective dans son dispositif (16, 28).

5. Procédé selon l'une des revendications précédentes, dans lequel
le stockage d'une définition de la partie de confiance respective comprend le stockage de sa clé publique dans le système auditif (10) ; et
la génération de l'approbation de partage respective comprend la signature d'une autorisation de partage avec une clé privée de la partie de confiance respective dans son dispositif (16, 28).

6. Procédé selon l'une des revendications précédentes, dans lequel
le stockage d'une définition de la partie de confiance respective comprend le stockage de son empreinte vocale dans le système auditif (10) ; et
la génération de l'approbation de partage respective comprend la transmission d'une phrase et/ou d'un mot clé d'approbation de partage prédéterminé(e), lu(e) et/ou prononcé(e) par la partie de confiance respective sous forme de signal audio à partir du dispositif (16, 28) de la partie de confiance respective à un dispositif de réception prédéterminé du système auditif (10) et la vérification du signal audio reçu en le comparant à l'empreinte vocale de cette partie de confiance.

7. Procédé selon l'une des revendications précédentes, dans lequel
le stockage d'une définition de la partie de confiance respective comprend le stockage de sa clé symétrique dans le système auditif (10) ;
la fourniture de la demande de partage au dispositif (16, 28) de la partie de confiance respective comprend le chiffrement d'une demande de partage avec la clé symétrique de cette partie de confiance et la transmission de cette demande de partage chiffrée à son dispositif (16, 28) ; et la génération de l'approbation de partage respective comprend le chiffrement d'une approbation de partage avec la clé symétrique de la partie de confiance respective dans son dispositif (16, 28).

8. Procédé selon l'une des revendications précédentes, dans lequel
des données de connexion pour la connexion à un serveur central prédéterminé (22) ainsi que sa clé publique sont sauvegardées dans le système auditif (10) ;
le stockage d'une définition de la partie de confiance respective comprend le stockage d'un numéro d'identification et/ou d'une clé publique de la partie de confiance respective dans ledit serveur central (22) ;
la fourniture de la demande de partage au dispositif (16, 28) de la partie de confiance respective comprend la transmission de la demande de partage à partir dudit serveur central (22) au dispositif (16, 28) de la partie de confiance respective ; et
la génération de l'approbation de partage respective comprend la transmission d'une autorisation de partage signée avec une clé privée de la partie de confiance respective à partir de son dispositif (16, 28) audit serveur central (22).

9. Procédé selon l'une des revendications précédentes,
dans lequel, avant d'être partagées, les données sont chiffrées avec une clé publique de la tierce partie (26) ou avec une clé symétrique de la tierce partie (26).

10. Procédé selon l'une des revendications précédentes, dans lequel l'étape de génération de la première et/ou de la seconde approbation de partage comprend l'obtention et la vérification d'une information contextuelle prédéfinie comprenant un ou plusieurs des éléments parmi :
des données concernant la tierce partie (26) ;
un type de données à partager ;
un moment auquel les données ont été collectées ;
un moment auquel la demande de partage a été générée ;
un moment auquel le partage peut se faire au plus tôt ;
un moment auquel le partage peut se faire au plus tard ;
un emplacement de l'appareil auditif à partir duquel le partage est autorisé, par exemple au domicile ;
un nombre et/ou un type de parties de confiance ;
un type de données biométriques requis ;
un type de réponse hors bande requis.

11. Procédé selon l'une des revendications précédentes,
dans lequel le système auditif (10) est configuré de telle sorte que l'étape de stockage d'une définition de la partie de confiance respective est limitée à être effectuée uniquement par une autorité de définition prédéterminée, dont les données de connexion et/ou la clé publique sont sauvegardées dans le système auditif (10).

12. Procédé selon l'une des revendications précédentes,
dans lequel le système auditif (10) est configuré de telle sorte que les étapes de stockage d'une définition de la partie de confiance respective et/ou de génération de l'approbation de partage respective sont au moins partiellement limitées à un canal de communication hors bande prédéterminé, qui est différent d'un canal de communication de données utilisé pour partager les données avec le dispositif tiers (24).

13. Programme d'ordinateur pour un partage protégé de données sauvegardées dans un système auditif (10), lequel système comprend au moins un appareil auditif (12, 14) utilisé par un utilisateur d'appareil auditif (20), avec un dispositif tiers (24), lequel programme, lorsqu'il est exécuté par un processeur, est conçu pour exécuter les étapes du procédé selon l'une des revendications précédentes.

14. Support lisible par ordinateur, sur lequel un programme informatique selon la revendication 13 est stocké.

15. Système auditif (10) comprenant un appareil auditif (12, 14), un dispositif (16) d'une première partie de confiance et un dispositif (28) d'une seconde partie de confiance, dans lequel l'appareil auditif (12, 14) comprend :
un microphone ;
un processeur pour le traitement d'un signal provenant du microphone ;
un dispositif de sortie pour délivrer le signal traité à une oreille d'un utilisateur d'appareil auditif ;
un émetteur-récepteur pour échanger des données avec le dispositif de la première et/ou de la seconde partie de confiance et/ou avec un dispositif d'utilisateur connecté et/ou avec un serveur central et/ou avec un autre appareil auditif,
dans lequel le système auditif (10) est conçu pour mettre en oeuvre le procédé selon l'une des revendications 1 à 12.
